(19) Europäisches Patentamt
European Patent Office
Office européen des brevets

(11) Veröffentlichungsnummer: **0 430 121 A1**

(12) **EUROPÄISCHE PATENTANMELDUNG**

(21) Anmeldenummer: 90122493.1

(22) Anmeldetag: 26.11.90

(51) Int. Cl.⁵: C07K 3/20

(30) Priorität: 27.11.89 DE 3939169

(43) Veröffentlichungstag der Anmeldung:
05.06.91 Patentblatt 91/23

(84) Benannte Vertragsstaaten:
AT BE CH DE DK ES FR GB GR IT LI LU NL SE
Patentblatt

(71) Anmelder: **BEHRINGWERKE**
**Aktiengesellschaft**
**Postfach 1140**
**W-3550 Marburg 1(DE)**

(72) Erfinder: **Römisch, Jürgen, Dr.**
**Am Kähnelplatz 6**
**W-3550 Marburg(DE)**
Erfinder: **Heimburger, Norbert, Prof.-Dr.**
**Sonnenhang 10**
**W-3550 Marburg(DE)**

(74) Vertreter: **Klein, Otto, Dr. et al**
**Hoechst AG Zentrale Patentabteilung**
**Postfach 80 03 20**
**W-6230 Frankfurt am Main 80(DE)**

(54) **Verfahren zur Reinigung von Lipocortinen.**

(57) Es wird ein Verfahren zur Gewinnung von Lipocortinen beschrieben, worin eine Lösung, die Lipocortine, besonders PP4-X, PAP III, p68, Anchorin II oder Lipocortin I oder II enthält, mit einem trägergebundenen Polyschwefelsäureester eines Saccharids oder einem trägergebundenen sulfatierten Zucker in Kontakt gebracht und die Lipocortine gewonnen werden.

EP 0 430 121 A1

## VERFAHREN ZUR REINIGUNG VON LIPOCORTINEN

Die Erfindung betrifft ein verfahren zur Reinigung von Lipocortinen, besonders der Proteine PP4-X, PAP III, p68, Anchorin II sowie der Lipocortine I und II.

Diese Proteine gehören einer Familie a von Proteinen an, die als Lipocortine, Calpactine oder Annexine bezeichnet werden. Sie konnten in vielen Organismen, so auch beim Menschen in den meisten Organen bzw. Zelltypen nachgewiesen werden. Die wichtigsten bisher bekannten funktionellen Eigenschaften dieser Proteine sind eine antiinflammatorische sowie antikoagulatorische Wirkung. Diese Proteinfamilie kann Einfluß auf entzündliche und oft damit zusammenhängende Gerinnungsstörungen wie die disseminierte intravasale Gerinnung (DIC) haben, diese abschwächen oder sogar neutralisieren.

Die bisherigen Verfahren zur schnellen Isolierung dieser Proteine (Tait, J.F. et al. (1988) Biochemistry 27, 6268-6276) sind für eine technische Isolierung dieser Proteine nicht geeignet.

Aufgabe der Erfindung war daher die Entwicklung eines Verfahrens zur Reinigung der Proteine PP4-X, PAP III, p68, Anchorin II sowie der Lipocortine I und II.

Es wurde nun überraschenderweise gefunden, daß diese Proteine in Gegenwart von Calcium Affinität zu Polyschwefelsäureestern von Sacchariden oder zu sulfatierten Zuckern aufweisen und an trägergebundene Polyschwefelsäureester von Sacchariden oder sulfatierte Zucker gebunden werden können, wobei der größte Teil an verunreinigenden Proteinen nicht adsorbiert wird. Die Adsorption der Lipocortine kann dabei in einer Säule oder auch im Batch erfolgen.

Gegenstand der Erfindung ist ein Verfahren zur Gewinnung von Lipocortinen, dadurch gekennzeichnet, daß eine Lösung, die ein Lipocortin oder mehrere Lipocortine enthält, in Gegenwart von Calciumionen mit einem trägergebundenen Polyschwefelsäureester eines Saccharids oder einem trägergebundenen sulfatierten Zucker in Kontakt gebracht, die überstehende Losung abgetrennt, das beladene Trägermaterial gegebenenfalls gewaschen und die Lipocortine einzeln, gruppenweise oder gesamt durch Erhöhung der Ionenstärke eluiert werden.

In einer Verfahrensweise wird eine 0.01-50 mg/ml eines Lipocortins enthaltende Lösung von pH 5.5-9.5, die 0.0001-0.1 mol/l Calciumionen beispielsweise in Form des Chlorids, Acetats oder Lactats enthält, mit einem trägergebundenen Polyschwefelsäurreester eines Saccharids oder einem sulfatierten Zucker, beispielsweise trägergebundenem Heparin, Dextransulfat, Heparansulfat, Chondroitinsulfat, Keratansulfat oder Dermatansulfat in Kontakt gebracht, die überstehende Lösung abgetrennt, das beladene Trägermaterial gegebenenfalls mit einem Puffer von pH 5.5-9.5, der 0.0001-0.1 mol/l Calcium enthält, gewaschen und das Lipocortin mittels eines linearen NaCl-, LiCl- oder KCl-Gradienten eluiert.

Falls Lipocortine gemischt in Lösung vorliegen, können sie auch gruppenweise oder gemeinsam eluiert werden.

Als wasserunlösliche Trägermatrix, an die ein Polyschwefelsäureester eines Saccharids oder ein sulfatierter Zucker kovalent gebunden werden, können z.B. unlösliches Dextran, Agarose, Acrylamid, ein aminofunktionalisiertes Polymeres und/oder Copolymere des Polyethylenglykols, Pentaerythrits und Methacrylats oder auch deren Kombinationen verwendet werden. Kupplung der Polyschwefelsäureester von Sacchariden oder der sulfatierten Zucker kann nach bekannten Methoden, z.B. an mittels CNBr oder Epoxid voraktiviertes Trägermaterial oder mittels Carbodiimid an aminofunktionalisiertes Trägermaterial, durchgeführt werden.

In einer bevorzugten Verfahrensweise wird eine ein Lipocortin enthaltende Lösung oder eine Lösung, die eine Kombination dieser Proteine oder alle diese Proteine enthält, in Gegenwart von 0.0002-0.03 mol/l Calcium bei pH 6-9 mit trägergebundenem Heparin oder Dextransulfat, bevorzugt aber mit einem Heparinaffinitätsharz, in Kontakt gebracht, die Mischung 3-300 min bei 4°-30°C inkubiert, die überstehende Lösung vom Trägerharz abgetrennt, das Adsorbens gegebenenfalls mit einer Lösung von pH 6-9, die 0.0002-0.03 mol/l Calcium enthält, gewaschen und die Proteine einzeln durch Erhöhung der Leitfähigkeit, vorzugsweise mittels eines linearen Salzgradienten, gruppenweise durch eine Stufenelution oder gesamt, vorzugsweise durch Erhöhung der Salzkonzentration auf 0.6-1.0 mol/l mittels NaCl, KCl, LiCl oder eines Salzes der Citronensäure eluiert.

Gegebenenfalls kann sich eine Nachreinigung einzelner oder aller Lipocortine anschließen. Dafür bieten sich folgende Möglichkeiten:

1. Rechromatographie an einem anderen trägergebundenen Polyschwefelsäureester eines Saccharids oder einem anderen sulfatierter Zucker.

2. Entfernung des Salzes bzw. der Calcium-Ionen, beispielsweise durch Dialyse, und Chromatographie mittels eines trägergebundenen Polyschwefelsäureesters eines Saccharids oder mittels eines sulfatierten Zuckers in Abwesenheit von Calcium bzw. Anwesenheit eines chelatbildenden Reagenzes wie EDTA, EGTA oder eines Salzes der Citronensäure oder Oxalsäure.

3. Ionenaustauschchromatographie, beispielsweise an Carboxymethyl-, DEAE-, QAE-RSepharose, RSephacel oder Cellulose (Firma Pharmacia, Schweden).

Das erfindungsgemäße Verfahren zeichnet sich dadurch aus, daß die Proteine PP4-X, PAP III, p68, Anchorin II und Lipocortin I und II in wenigen Schritten in sehr hoher Reinheit und Ausbeute auch im großtechnischen Maßstab isoliert werden können.

Zur großtechnischen Isolierung dieser Lipocortine bietet sich als Rohstoffquelle vor allem humane Plazenta an, die in ausreichenden Mengen verfügbar ist und diese Proteine in großen Mengen enthält. Für die Reinigung von Anchorin II bieten sich vor allem die dieses Protein enthaltende Fibroblasten besonders an.

Erläuterung der Abkürzungen:

EDTA: Ethylendiamintetraessigsäure

EGTA: Bis(aminoethyl)-glycolether-N,N,N',N'- tetraessigsäure

DEAE: Diethylaminoethyl

QAE: Quartäres Aminoethyl

PP4-X: Plazentares Protein

PAP: Plazentares Antikoagulatorisches Protein

Die Erfindung soll durch die folgenden Beispiele erläutert werden:

Beispiel 1

36 kg reife, menschliche Plazenta wurden zerkleinert, mit 0.9 g/100 ml NaCl-Lösung mehrmals gewaschen und anschließend lyophilisiert. Das Lyophilisat (3 kg) wurde mit 50 l einer Lösung aus 0.02 mol/l Tris/HCl, pH 7.5, 0.15 mol/l NaCl und 0.1 M Tri-Na-Citrat extrahiert, mit Ammoniumsulfat (33 % Sättigung) versetzt und mit 4 l Phenylsepharose im Batch-Verfahren inkubiert. Nach Waschen des Harzes wurden die Lipocortine mit Wasser im Stoß eluiert, durch Zusatz von Ammoniumsulfat auf 80 % Sättigung gefällt, durch Zentrifugation pelletiert, in einer Lösung aus 0.02 mol/l Tris/HCl, pH 8.0 (Puffer A) aufgenommen und gegen den selben Puffer dialysiert. Nach Zusatz von $CaCl_2$ bis zu einer Endkonzentration von 0.05 mol/l wurde das Dialysat im Batch-Verfahren mit 1 l einer Heparin-RSepharose versetzt, 60 min bei Raumtemperatur gerührt und die überstehende Lösung abgetrennt. Das Adsorbens wurde danach mit Puffer A, unter Zusatz von 0.005 mol/l $CaCl_2$, gewaschen und mit 0.06 mol/l NaCl voreluiert. Die Proteine PP4-X, PAP III, p68 sowie Lipocortin I und II wurden zusammen im Stoß mit 0.8 mol/l NaCl eluiert.

Nach Dialyse des Eluates gegen Puffer A wurden restliche Verunreinigungen dadurch entfernt, daß nach Zugabe von EDTA auf eine Endkonzentration von 0.001 mol/l die proteinhaltige Lösung mit 200 ml einer Heparin-RSepharose, äquilibriert in 0.02 mol/l Tris/HCl, pH 8.0, 0.001 mol/l EDTA, in Kontakt gebracht wurde, für 60 min bei Raumtemperatur gerührt und die überstehende, die Lipocortin-Proteine enthaltende Lösung abgetrennt wurde. Die auf diese Weise gereinigten Lipocortin-Proteine zeigten in der SDS-Polyacrylamid-Gelelektrophorese (PAGE) Banden mit Molekulargewichten von 33 kD (PP4-X), 34 kD (PAP III), 68 kD (p68), 36 kD (Lipocortin I) sowie 37 kD (Lipocortin II, schwere Kette) und 10 kD (leichte Kette). Isolierung der einzelnen Proteine wurde mittels Ionenaustauschchromatographie durchgeführt, wie in Beispiel 2 beschrieben.

Die Ausbeuten für die einzelnen Proteine lagen zwischen 70 und 90%, bezogen auf das Phenyl-RSepharose-Eluat.

Beispiel 2

Eine PP4-X, PAP III, p68 sowie Lipocortin I und II enthaltende Lösung wurde, wie in Beispiel 1 beschrieben, mit Heparin-RSepharose in Kontakt gebracht und das Adsorbens danach gewaschen. Durch stufenelution wurden PP4-X (0.35 mol/l NaCl) und PAP III (0.48 mol/l Nacl) getrennt eluiert. Lipocortin I, II und p68 konnten mit 0.8 mol/l Nacl zusammen eluiert werden. PP4-X bzw. PAP III oder p68, Lipocortin I und II wurden von restlichen Verunreinigungen, wie in Beispiel 1 beschrieben, befreit. Weitere Auftrennung der durch 0.8 mol/l Nacl eluierten Proteine erfolgte durch Ionenaustauschchromatographie:

Die p68, Lipocortin I und II enthaltende Lösung wurde gegen Puffer A dialysiert und auf eine DEAE-RSepharoseSäule, äquilibriert mit Puffer A, gepumpt. Adsorbiertes Protein p68 wurde mittels eines NaCl-Gradienten eluiert. Die bei dieser Chromatographie nicht adsorbierten Proteine Lipocortin I und II wurden gegen einen Puffer aus 0.01 mol/l Imidazol/HCl, pH 6.0, dialysiert, unter Rühren mit Carboxymethyl-Cellulose in Kontakt gebracht, danach das Adsorbens gewaschen und in eine Säule gepackt. Lipocortin I und II wurden getrennt durch Anlegen eines linearen Salz-Gradienten von 0-0.7 mol/l NaCl eluiert. Die so isolierten Lipocortin-Proteine hatten eine Reinheit von >95% und zeigten in der SDS-PAGE jeweils die in Beispiel 1 beschriebenen Molekulargewichte.

**Ansprüche**

1. Verfahren zur Gewinnung von Lipocortinen, dadurch gekennzeichnet, daß eine Lösung, die ein Lipocortin oder mehrere Lipocortine enthält, in Gegenwart von Calciumionen mit einem trägergebundenen Polyschwefelsäureester eines Saccharids oder einem trägergebundenen sulfatierten Zucker in Kontakt gebracht, die überstehende Lösung abgetrennt, das beladene Trägermaterial gegebenenfalls gewaschen und die Lipocortine einzeln, gruppenweise oder gesamt durch Erhöhung der Ionenstärke eluiert werden.

2. Verfahren nach Anspruch 1, dadurch gekennzeichnet, daß die Lipocortine PP4-X, PAP III, p68, Anchorin II oder Lipocortin I oder II sind.

3. Verfahren nach Anspruch 1, dadurch gekennzeichnet, daß die Lipocortine in Abwesenheit von Calciumionen oder in Anwesenheit eines Überschusses eines chelatbildenden Reagenzes durch Adsorption restlicher Verunreinigungen an einen Polyschwefelsäureester eines Saccharids oder einen sulfatierten Zucker und Abtrennung der lipocortinhaltigen Lösung nachgereinigt werden.

4. Verfahren nach mindestens einem der Ansprüche 1 und 3, dadurch gekennzeichnet, daß das Trägermaterial unlösliches Dextran, Agarose, Polyacrylamid, ein aminofunktionalisiertes unlösliches Polymeres oder ein Copolymeres des Polyethylenglykols, Pentaerythrits und Methacrylats oder eine Kombination von diesen ist.

5. Verfahren nach mindestens einem der Ansprüche 1 und 3, dadurch gekennzeichnet, daß der Polyschwefelsäureester eines Saccharides Heparin, Dextransulfat, Heparansulfat, Chondroitinsulfat, Keratansulfat oder Dermatansulfat ist.

6. Verfahren nach mindestens einem der Ansprüche 1 und 3, dadurch gekennzeichnet, daß trägergebundenes Heparin oder Dextransulfat verwendet wird.

7. Verfahren nach mindestens einem der Ansprüche 1 und 3, dadurch gekennzeichnet, daß trägergebundenes Heparin verwendet wird.

8. Verfahren nach Anspruch 1, dadurch gekennzeichnet, daß das Verfahren in einem Puffer von pH 5.5-9.5, der 0.001-0.1 mol/l Calciumionen enthält, ausgeführt wird.

9. Verfahren nach Anspruch 2, dadurch gekennzeichnet, daß als chelatbildendes Reagenz EDTA, EGTA, ein Salz der Citronensäure oder Oxalsäure oder eine Kombination daraus verwendet wird.

10. Verfahren nach Anspruch 2, dadurch gekennzeichnet, daß als chelatbildendes Reagenz EDTA oder EGTA verwendet wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 301 374   (BEHRINGWERKE)<br>* das ganze Dokument * | 1-8 | C 07 K 3/20 |
| A | PROCEEDINGS OF THE NATIONAL ACADEMY OF SCIENCES OF USA. vol. 85, Juni 1988, WASHINGTON US Seiten 3708 - 3712; Ulrich G.et al: "Characterization of cDNA encoding human anticoagulant protein......"<br>* Seiten 3710 - 3711 * | 1-10 | |
| A | JOURNAL OF BIOCHEMISTRY. vol. 106, no. 1, Juli 1989, TOKYO JP Seiten 43 - 49; Akio I.et al: "Structure and expression of cDNA for calphobindin II,a human....."<br>* Seite 47 * | 1-10 | |
| A | EP-A-0 315 081   (BEHRINWERKE)<br>* Seite 2 * | 3,9-10 | |
| X,P | BIOLOGICAL CHEMISTRY HOPPE-SEYLER vol. 371, no. 5, Mai 1990, BERLIN Seiten 383 - 388; Roemisch,J.et al: "Purification and characterization of six annexins from human placenta"<br>* das ganze Dokument * | 1-10 | **RECHERCHIERTE SACHGEBIETE (Int. Cl.5)**<br><br>A 61 K<br>C 12 N<br>C 07 K |

Der vorliegende Recherchenbericht wurde für alle Patentansprüche erstellt

| Recherchenort | Abschlußdatum der Recherche | Prüfer |
|---|---|---|
| Den Haag | 06 März 91 | FERNANDEZ Y BRANAS F |